# EUROPEAN PATENT SPECIFICATION

(11) **EP 2 906 953 B1**
(45) Date of publication and mention of the grant of the patent: **06.12.2017**
(21) Application number: 13780067.8
(22) Date of filing: 14.10.2013
(51) Int. Cl.: G01N 33/574

(54) **NOVEL BIOMARKERS FOR SUB-TYPING PANCREATIC DUCTAL ADENOCARCINOMA**
NEUARTIGE BIOMARKER ZUR SUBTYPISIERUNG VON DUKTALEM PANKREASADENOKARZINOM
NOUVEAUX BIOMARQUEURS POUR LE SOUS-TYPAGE D'UN ADÉNOCARCINOME CANALAIRE DU PANCRÉAS

(30) Priority: 12.10.2012 EP 12007128
(43) Date of publication of application: 19.08.2015
(73) Proprietor: HI-STEM gGmbH Im Deutschen Krebsforschungszentrum DKFZ, 69120 Heidelberg (DE)
(72) Inventor: EISEN, Christian, Thomas, 69120 Heidelberg (DE); TRUMPP, Andreas, 69115 Heidelberg (DE); SPRICK, Martin, Ronald, 69131 Heidelberg (DE)
(74) Representative: Virnekäs, Bernhard
(86) International application number: PCT/EP2013/003085
(87) International publication number: WO 2014/056626

(56) References cited:
- Ironman: "Microsoft Word - 050812_Complete_PhD_final.docx", , 6 August 2012 (2012-08-06), XP055095160, Retrieved from the Internet: URL:http://archiv.ub.uni-heidelberg.de/vol ltextserver/14021/1/EISEN_Complete_PhD.pdf [retrieved on 2014-01-08]
- ERIC A COLLISSON ET AL: "Subtypes of pancreatic ductal adenocarcinoma and their differing responses to therapy", NATURE MEDICINE, vol. 17, no. 4, 1 April 2011 (2011-04-01), pages 500-503, XP055089296, ISSN: 1078-8956, DOI: 10.1038/nm.2344
- Alexey Aleshin ET AL: "SRC: a century of science brought to the clinic", Neoplasia (New York, N.Y.), 1 August 2010 (2010-08-01), pages 599-607, XP055104230, Canada DOI: 10.1593/neo.10328 Retrieved from the Internet: URL:http://www.ncbi.nlm.nih.gov/pubmed/206 89754 [retrieved on 2014-02-25]
- DUNCAN T ODOM ET AL: "Tissue-specific transcriptional regulation has diverged significantly between human and mouse", NATURE GENETICS, vol. 39, no. 6, 1 June 2007 (2007-06-01), pages 730-732, XP055104365, ISSN: 1061-4036, DOI: 10.1038/ng2047
- LAURA PELLETIER ET AL: "HNF1Î+- inhibition triggers epithelial-mesenchymal transition in human liver cancer cell lines", BMC CANCER, BIOMED CENTRAL, LONDON, GB, vol. 11, no. 1, 5 October 2011 (2011-10-05), page 427, XP021110261, ISSN: 1471-2407, DOI: 10.1186/1471-2407-11-427
- TOUKAP A NZEUSSEU ET AL: "Identification of distinct gene expression profiles in the synovium of patients with systemic lupus erythematosus", ARTHRITIS & RHEUMATISM, J.B. LIPPINCOTT CO, vol. 56, no. 5, 1 May 2007 (2007-05-01), pages 1579-1588, XP002612544, ISSN: 0004-3591, DOI: 10.1002/ART.22578 [retrieved on 2007-04-27]
- BIOLOGICAL ABSTRACTS, vol. unknown 7 September 2011 (2011-09-07), Philadelphia, PA, US, abstract no.: 274, Eisen: "An advanced culture system for pancreatic cancer reveals tumor heterogeneity and facilitates stratification of novel diagnostic and therapeutic targets",
- EISEN: "Subtype-specific Models of Pancreatic Ductal Adenocarcinoma Reveal Signaling Pathways That Can Be Exploited Therapeutically", EUROPEAN JOURNAL OF CANCER, vol. 48, no. Suppl. 5, July 2012 (2012-07) , page S75, 22ND BIENNIAL CONGRESS OF THE EUROPEAN-ASSOCIATION-FOR-CANCER-RESEARCH; BARCELONA, SPAIN; JULY 07 -10, 2012

## Description

### FIELD OF THE INVENTION

This invention relates to novel approaches for the identification and stratification of subtypes of pancreatic ductal adenocarcinoma (PDAC), in particular to novel PDAC subtype-specific markers, and to diagnostic kits comprising reagents for detecting said markers.

### BACKGROUND OF THE INVENTION

Personalized oncology has the potential to revolutionize the way cancer patients will be treated in the future. Different entities of cancer can be divided into subclasses based on molecular differences, including the specific activation of signaling pathways that often determine therapy response and clinical outcome. For various cancer entities including breast, lung and colon cancer, the identification of such subtypes and the possibility to stratify patients into cohorts has already been translated into clinical practice to treat patients in a subtype-specific manner.

PDAC is the most frequent pancreatic cancer and the fourth cause of cancer death in the United States and Europe. Most patients die within 12 months, and only 2% survive five years after prognosis. Little progress in the treatment of PDAC has been made since the approval of Gemcitabine in 2000 and Erlotinib in 2005. Moreover, recent trials with targeted therapies have shown limited or no benefit.

PDAC is still classified as a single cancer entity and is clinically treated as such. However, the existence of three PDAC subtypes termed classical, quasi-mesenchymal and exocrine-like has recently been suggested (Collisson, E. A., Sadanandam, A., Olson, P., Gibb, W. J., Truitt, M., Gu, S. D., Cooc, J., Weinkle, J., Kim, G. E., Jakkula, L., et al. (2011). Subtypes of pancreatic ductal adenocarcinoma and their differing responses to therapy. Nat Med 17, 500-U140). The identification of these subtypes was based on comparative gene expression analysis in micro-dissected epithelial cells form patient specimens.

The existence of PDAC subtypes raises the possibility of inter-subtype specific differences regarding the sensitivity to therapeutic agents. The successful establishment of individualized therapy approaches for PDAC depends on novel biomarkers that allow for reliable and accurate attribution of PDAC specimen to a specific PDAC subtype. Such novel subtype-specific biomarkers would be useful for quick and reliable patient stratification for individualized therapy of PDAC. The great need for improved therapy approaches for PDAC based on patient stratification has not been met so far.

### OBJECTS OF THE INVENTION

It was thus an object of the invention to provide novel biomarkers for PDAC, in particular of the classical, the quasi-mesenchymal, and the exocrine-like subtype that allow for reliable and accurate attribution of PDAC specimen to a specific PDAC subtype. Such novel PDAC subtype-specific biomarkers would satisfy the great need for quick and reliable patient stratification to greatly improve prognostic evaluation and introduction of novel PDAC treatment approaches exploiting subtype-specific drug vulnerabilities.

### SUMMARY OF THE INVENTION

Surprisingly it has been found that novel biomarkers specific for subtypes of PDAC can be identified. Furthermore, it was surprisingly found that the novel PDAC subtype-specific biomarkers of the present invention allow for reliable and accurate attribution of PDAC specimen to a specific PDAC subtype. The novel subtype-specific biomarkers of the present invention might be useful to greatly improve prognostic evaluation of patients and PDAC treatment approaches by exploitation of subtype-specific drug vulnerabilities. Quick and reliable PDAC patient stratification has been rendered possible for the first time utilizing the novel biomarkers of the present invention.

Thus, in one aspect, the present invention relates to an *in vitro* method for the subtype-specific detection of PDAC cells in a tumor sample characterized by the step of detecting expression of subtype-specific markers, either as proteins or as oligonucleotides encoding such proteins, wherein keratin 81 and vimentin are detected as markers for PDAC cells of the quasi-mesenchymal subtype, wherein (i) one or more transcription factors selected from HNF-1A, HNF-1B, FOXA2 (HNF3B), FOXA3 (HNF3G), HNF4G, and ONECUT1 (HNF6), (ii) one or more target genes regulated by HNF-1A, particularly the HNF-1A target genes listed in Table 2, and (iii) cadherin17 (CDH17) are detected as markers for cells of the exocrine-like subtype, and wherein PDAC cells of the classical subtype are characterized by the absence of (i) keratin 81 and/or vimentin, and (ii) HNF-1A and/or HNF-1B.

In another aspect, the disclosure relates to an *in vitro* method for the subtype-specific detection of PDAC cells in a tumor sample, characterized by the step of analyzing the Src inhibitor sensitivity signature in a gene expression profile.

In another aspect, the present invention relates to a kit for the subtype-specific detection of PDAC cells comprising at least two reagents specific for PDAC subtype-specific markers selected from the list of: (i) keratin 81, (ii) vimentin, (iii) one or more transcription factors selected from HNF-1A, HNF-1B, FOXA2 (HNF3B), FOXA3 (HNF3G), HNF4G, and ONECUT1 (HNF6), (iv) one or more target genes regulated by HNF-1A, particularly the HNF-1A target genes listed in Table 2, and (v) cadherin17 (CDH17), wherein said reagents are selected from the list of: detection antibodies and oligonucleotide probes, and optionally further comprising at least one reagent for the detection of at least one of said reagents.

In another aspect, the disclosure relates to a kit for the subtype-specific detection of Src inhibitor-sensitive PDAC cells comprising oligonucleotide probes for the identification of a Src inhibitor sensitivity signature.

In another aspect, the present invention relates to a method of stratifying patients suffering from PDAC into cohorts, the method comprising the steps of: (a) *in vitro* determination of (i) the expression and/or the presence of (i) keratin 81, (ii) vimentin, (iii) one or more transcription factors selected from HNF-1A, HNF-1 B, FOXA2 (HNF3B), FOXA3 (HNF3G), HNF4G, and ONECUT1 (HNF6), (iv) one or more HNF-1A target genes, particularly the HNF-1A target genes listed in Table 2, and/or (v) cadherin17 (CDH17), (b) identifying the PDAC subtype based on the expression and/or the presence of the selected proteins, and (c) stratifying PDAC patients into prognostic and/or drug treatment cohorts based on the PDAC subtype they are suffering from.

In another aspect, the disclosure relates to a method of stratifying patients suffering from PDAC into cohorts, the method comprising the steps of: (a) *in vitro* gene expression analysis for determination of the Src inhibitor sensitivity signature in cells of a patient sample containing pancreatic tissue, (b) identifying the PDAC subtype based on the gene expression analysis, and (c) stratifying PDAC patients into prognostic and/or drug treatment cohorts based on the PDAC subtype they are suffering from.

### FIGURES

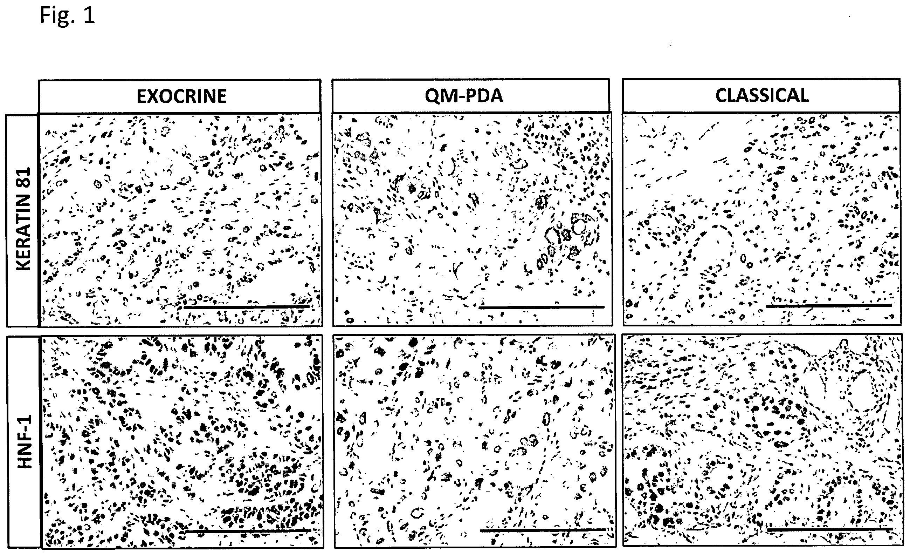

**Figure 1** shows the identification of PDAC-subtype specific markers. Immunohistochemical staining of representative tumors of the different subtypes (QM-PDAC - PACO 7 DT, Exocrine-like - PACO 3 DT, Classical - PACO 2 DT) for keratin 81 (top row) and HNF-1 A (bottom row) (scale bars 200 µm).

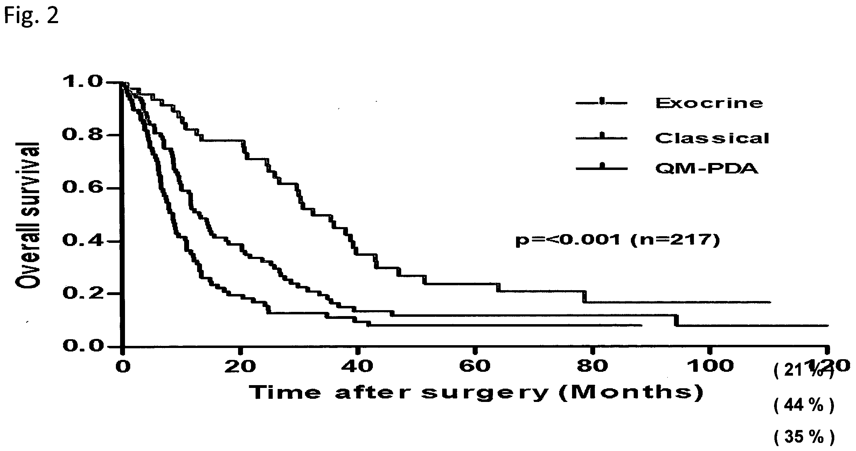

**Figure 2** demonstrates patient stratification by the newly identified markers. Patients were stratified according to the method of the invention and their subtype correlated with mean survival. A highly significant correlation was found for the respective subtypes and patient survival.
**Figure 3** shows an overview of the total number of cases having been examined, their subtype assignment and the mean survival of the three groups.
**Figure 4** details the patient characteristics included in the tissue microarray.

### DETAILED DESCRIPTION OF THE INVENTION

The present invention may be understood more readily by reference to the following detailed description of the invention and the examples included therein.

In one aspect, the present invention relates to an *in vitro* method for the subtype-specific detection of PDAC cells in a tumor sample characterized by the step of detecting expression of subtype-specific markers, either as proteins or as oligonucleotides encoding such proteins, wherein keratin 81 and vimentin are detected as markers for PDAC cells of the quasi-mesenchymal subtype, wherein (i) one or more transcription factors selected from HNF-1A, HNF-1B, FOXA2 (HNF3B), FOXA3 (HNF3G), HNF4G, and ONECUT1 (HNF6), (ii) one or more target genes regulated by HNF-1A, particularly the HNF-1A target genes listed in Table 2, and (iii) cadherin17 (CDH17) are detected as markers for cells of the exocrine-like subtype, and wherein PDAC cells of the classical subtype are characterized by the absence of (i) keratin 81 and/or vimentin, and (ii) HNF-1A and/or HNF-1B.

In the context of the present invention, "PDAC" refers to pancreatic ductal adenocarcinoma, the most common type of pancreatic cancer, accounting for 95% of these tumors, arising within the exocrine component of the pancreas. It is typically characterized by moderately to poorly differentiated glandular structures on microscopic examination.

In the context of the present invention, "pancreatic cancer" refers to a cancer originating from transformed cells arising in tissues forming the pancreas.

In the context of the present invention, the terms "classical", "quasi-mesenchymal", and "exocrine-like subtype of PDAC" refer to the PDAC subtypes as identified by Collisson et al. (loc. cit.) based on their gene expression profiles. In this study, a 62-gene panel was devised that enables classification of tumor samples into one of the three subtypes.

Tumors of the quasi-mesenchymal PDAC subtype give rise to poorly differentiated tumors characterized by abundant mitoses with abnormal spindle formation and cells with large cytoplasm and anisomorphic (i.e. of abnormal morphology) to pleomorphic (i.e. variable in shape) nuclei.

The classical or the exocrine-like PDAC subtype gives rise to tumors with a more differentiated growth pattern of medium-sized neoplastic duct-structures with only moderate variation in nuclear size and chromatin structure.

The present inventors surprisingly identified that cells of PDAC of the quasi-mesenchymal subtype show specific expression of the type-II keratin 81 and/or vimentin. While expression of keratin 81 is usually restricted to normal hair follicles, a correlation has been reported between keratin 81 expression and (a) pulmonary metastasis in breast cancer and (b) the risk of recurrence in lung cancer. Vimentin expression can be found in stroma cells, so that any marker analysis in accordance with the present invention has ensure that the cells to be tested are tumor cells, and do not comprise stroma cells.

In the context of the present invention, the term "specific expression" refers to the detection of a protein or a transcript in a sample compared to one or more comparator samples. The expression of an investigated marker is considered specific to a sample if of 500 analyzed tumor cells at least 1 tumor cell shows a signal above that observed with an unspecific control antibody and in the comparator sample or comparator samples no positive signal for the investigated marker can be detected.

In the context of the present invention, the term "specific expression" for analysis can also refer to the detection of the amount of a specific RNA-transcript in the total sample. The relative amount of the mRNA can be determined quantitatively (by e.g. qRT-PCR) by comparing it to one or more suited standards (e.g. the housekeeping genes beta-actin or GAPDH). Alternatively, the expression can be determined by comparison to other tumor samples or normal, non-cancerous, tissue. If the relative expression of the transcript is greater than a previously determined cutoff (e.g.1.5 fold, 2 fold, 5 fold or 10 fold) the expression is considered to be specific for the sample.

In the context of the present invention, the term "absence" refers to the percentage of marker-positive tumor cells in a sample (determined as described in Section [0026]). If in 500 analyzed tumor cells of a sample no cell with a signal for the investigated marker is detected, the marker is considered to be 'absent' in the sample. Alternatively, if the level of a transcript (determined as described in Section [0027]) is below a previously determined cut-off, the transcript is considered to be 'absent' from the sample.

Furthermore, the present inventors surprisingly identified (i) transcription factors selected from HNF-1A, HNF-1B, FOXA2 (HNF3B), FOXA3 (HNF3G), HNF4G, and ONECUT1 (HNF6), (ii) HNF-1A target genes, particularly HNF-1A target genes listed in Table 2, and (iii) cadherin17 (CDH17) are specifically expressed in PDAC cells of the exocrine-like subtype. HNF-1A and HNF-1B are transcription factors, which are expressed in the liver and have previously been described in both endo- and exocrine cells of the developing pancreas, while they are not expressed in the adult normal pancreas. Further, expression of cadherin17 (CDH17) has also been found to be specific for the exocrine-like subtype.

Finally, the present inventors surprisingly identified that cells of the classical PDAC subtype are characterized by the absence of (i) keratin 81 and/or, vimentin, and (ii) HNF-1A and/or HNF-1B, and optionally, the additional absence of one or more of the transcription factors HNF-1A, HNF-1B, FOXA2 (HNF3B), FOXA3 (HNF3G), HNF4G, and ONECUT1 (HNF6), HNF-1A target genes, particularly HNF-1A target genes listed in Table 2, and cadherin17 (CDH17).

In a particular embodiment, PDAC of the classical subtype are identified by determining the absence of specific expression of the biomarkers keratin 81 and HNF-1A.

In particular embodiments, PDAC of the classical subtype are identified by determining the absence of specific expression of one or more additional biomarkers selected from (i) transcription factors selected from HNF-1A, HNF-1B, FOXA2 (HNF3B), FOXA3 (HNF3G), HNF4G, and ONECUT1 (HNF6), (ii) one or more target genes regulated by HNF-1A, particularly the HNF-1A target genes listed in Table 2, and (iii) cadherin17 (CDH17).

In one particular aspect, the present invention relates to an *in vitro* method for the subtype-specific detection of PDAC cells in a tumor sample characterized by the step of detecting expression of subtype-specific markers, either as proteins or as oligonucleotides encoding such proteins, wherein keratin 81 is the marker for PDAC cells of the quasi-mesenchymal subtype, wherein (i) HNF-1A is the marker for cells of the exocrine-like subtype, and wherein PDAC cells of the classical subtype are characterized by the absence of keratin 81 and HNF-1A.

In particular embodiments, said detection is based on binding of a detection antibody to an epitope of one of said subtype-specific markers.

In the context of the present invention, the term "epitope" refers to the part of an antigen that is recognized by an antibody.

In particular embodiments, the *in vitro* process for the subtype-specific detection of PDAC cells comprises the steps of (a) bringing said sample into contact with a detection antibody specific for one of said subtype-specific markers under conditions that allow binding of the detection antibody to said epitope, (b) removal of excess detection antibody molecules, and (c) detection of bound detection antibody in said sample.

In the context of the present invention, the term "comprises" or "comprising" means "including, but not limited to". The term is intended to be open-ended, to specify the presence of any stated features, elements, integers, steps or components, but not to preclude the presence or addition of one or more other features, elements, integers, steps, components, or groups thereof. The term "comprising" thus includes the more restrictive terms "consisting of" and "consisting essentially of".

In particular embodiments, the detection antibody is a monoclonal antibody, a polyclonal antibody, a recombinantly produced antibody, including a recombinantly produced chimeric or humanized antibody, or a fully synthetic antibody. In particular embodiments, the antibody is a full immunoglobulin, including an IgA, IgD, Ige, IgG, and IgM, particularly an IgA or IgG. In particular embodiments, the antibody is a functional fragment of an antibody, including a Fab fragment, a F(ab')2 fragment, an Fv fragment, and a single chain Fv fragment (scFv).

In the context of the present invention, the term "monoclonal antibody" refers to a monospecific antibody that is produced by an immortalized unique parent immune cell.

In the context of the present invention, the term "polyclonal antibodies" refer to antibodies that are obtained from different B cell resources. They are a combination of immunoglobulin molecules secreted against a specific antigen, identifying different epitopes.

In the context of the present invention, the term "chimeric antibody" refers to an antibody made by combining genetic material from a non-human source with human genetic material, particular antibodies obtained by combining non-human variable regions from a target-specific antibody of a non-human species with human antibody constant domains. Such chimeric antibodies are generally around two thirds human, reducing the risk of a reaction to foreign antibodies from a non-human animal when they are used in therapeutic treatments.

In the context of the present invention, the term "humanized antibody" refers to a particular form of a chimeric antibody, wherein CDR regions from a target-specific antibody of a non-human species are inserted into a human antibody variable domain framework, particularly wherein certain residues of the human framework are back-mutated to residues resembling the non-human species in order to provide appropriate structural support for the three-dimensional arrangement of the binding CDR loops. Such CDR-grafted or humanized antibodies are thus composed to an even larger extent of human sequences than chimeric antibodies described above.

In the context of the present invention, the term "functional fragment of an antibody" refers to an antibody-based protein that retains the property of being able to specifically bind to an antigen. In certain embodiments, such fragment comprises at least the CDR1, CDR2 and CDR3 regions of a variable heavy chain (VH chain). In certain embodiments, such fragment comprises a fully VH chain (CDR1, CDR2 and CDR3 regions plus framework regions FR1, FR2, FR3 and FR4). In certain embodiments, such fragment comprises at least the CDR1, CDR2 and CDR3 regions of a variable light chain (VL chain). In certain embodiments, such fragment comprises a fully VL chain (CDR1, CDR2 and CDR3 regions plus framework regions FR1, FR2, FR3 and FR4). In certain embodiments, such fragment comprises at least the CDR1, CDR2 and CDR3 regions of both VH and VL chain (VH chain). In certain embodiments, such fragment comprises both a full VH and a full VL chain.

In the context of the present invention, the term Fab fragment relates to functional fragment of an antibody that is composed of one constant and one variable domain of each of the heavy and the light chain. The two variable domains bind the epitope on their specific antigens.

In the context of the present invention, the term F(ab')2 fragment relates to two identical Fab fragments connected via a hinge region.

In the context of the present invention, the term "single chain antibody" or "single-chain variable fragment (scFv)" refers to a fusion protein of the variable regions of the heavy (VH) and light chains (VL) of immunoglobulins, connected with a short linker peptide of ten to about 25 amino acids. The linker is usually rich in glycine for flexibility, as well as serine or threonine for solubility, and can either connect the N-terminus of the VH with the C-terminus of the VL, or vice versa. This protein retains the specificity of the original immunoglobulin, despite removal of the constant regions and the introduction of the linker.

In particular embodiments, detection antibody binding is detected by incubation with a secondary antibody, particularly a labeled secondary antibody.

In particular embodiments, the secondary antibody is labeled with a marker molecule.

In particular embodiments, the secondary antibody is labeled with a fluorescent dye, a biologically active enzymatic label, a radioactive label, a nuclear magnetic resonance active label, a luminescent label or a chromophoric label.

In particular embodiments, the radioactive label is selected from the group of ³²P, ¹²⁵I ³H, ¹⁴C, ¹⁸⁸Rh, ¹³¹I, ⁹⁹mTc and ¹¹¹In .

In a particular embodiment, antibody binding is detected by immunohistological staining.

In particular embodiments, said detection is based on determining mRNA encoding one of said subtype-specific markers, particularly by quantitative real-time polymerase chain reaction (qRT-PCR).

In a particular embodiment, the sample to be tested contains pancreatic tissue.

In particular embodiments, said sample is obtained from a mammal, particularly a human.

In another aspect, the disclosure relates to an *in vitro* method for the subtype-specific detection of PDAC cells in a tumor sample, characterized by the step of analyzing the Src inhibitor sensitivity signature in a gene expression profile.

In the context of the disclosure, "Src" relates to a protein (also called c-Src for "cellular Src"), which is a tyrosine kinase encoded by the proto-oncogene SRC, which is frequently overexpressed and highly activated in malignancies. Src is a member of a kinase family (the so-called "Src family"). Additional members of that family are: Lyn, Fyn, Lck, Hck, Fgr, Blk, Yrk und c-Yes.

Surprisingly, the present inventors have found that PDAC cells can be separated into a Src inhibitor sensitivity predictor-positive PDAC subtype and a Src inhibitor sensitivity predictor-negative PDAC subtype.

In the context of the disclosure, the term "Src inhibitor sensitivity signature" refers to the gene expression signature characteristic of cells which are sensitive to Src inhibitors, as established in accordance with Example 2.

In the context of the disclosure, the term "Src inhibitor sensitivity predictor-positive PDAC subtype" refers to a subtype that is characterized by tumor cells scoring positive in a gene expression analysis using a list of gene-expression values as input for the GSEA algorithm (Subramanian, A., Tamayo, P., Mootha, V. K., Mukherjee, S., Ebert, B. L., Gillette, M. A., Paulovich, A., Pomeroy, S. L., Golub, T. R., Lander, E. S., and Mesirov, J. P. (2005). Gene set enrichment analysis: a knowledge-based approach for interpreting genome-wide expression profiles. Proc Natl Acad Sci U S A 102, 15545-15550). In the context of the disclosure, "scoring positive" means having a false discovery rate ("FDR") of less than 0.200. Conversely, the term "Src inhibitor sensitivity predictor-negative PDAC subtype" refers to a subtype that is characterized by tumor cells scoring negative in such gene expression analysis with an FDR of 0.200 or higher.

In a particular example, the gene expression profile is based on the set of 30 genes as shown in Table 1, particularly wherein the gene expression profile is established as described in Example 2.

The present inventors have found that cells of the classical PDAC subtype, and certain cells of the quasi-mesenchymal PDAC subtype belong to the Src inhibitor sensitivity predictor-positive PDAC subtype, and such cells are sensitive to Src inhibitors, while the remaining cells of the quasi-mesenchymal PDAC subtype as well as cells of the exocrine-like subtype belong to the Src inhibitor sensitivity predictor-negative PDAC subtype, and such cells are resistant to Src inhibitors.

In another aspect, the present invention relates to a kit for the subtype-specific detection of PDAC cells comprising at least two reagents specific for PDAC subtype-specific markers selected from the list of: (i) keratin 81, (ii) vimentin, (iii) one or more transcription factors selected from HNF-1A, HNF-1B, FOXA2 (HNF3B), FOXA3 (HNF3G), HNF4G, and ONECUT1 (HNF6), (iv) one or more HNF-1A target genes, particularly HNF-1A target genes listed in Table 2, and (v) cadherin17 (CDH17), wherein said reagents are selected from the list of: detection antibodies and oligonucleotide probes, and optionally further comprising at least one reagent for the detection of at least one of said reagents.

In a particular embodiment, said kit comprises (i) at least one reagent specific for the quasi-mesenchymal PDAC subtype selected from the list of: keratin 81 and vimentin, particularly keratin 81, and (ii) at least one reagent specific for the exocrine-like PDAC subtype selected from the list of: HNF-1A and HNF-1B, particularly HNF-1A.

In another aspect, the present disclosure relates to a kit for the subtype-specific detection of Src inhibitor-sensitive PDAC cells comprising oligonucleotide probes for the identification of a Src inhibitor sensitivity signature.

In a particular example, the kit comprises oligonucleotide probes representing the 30 genes shown in Table 1.

In a particular example, the oligonucleotides are present in an oligonucleotide array, particularly an array on a gene chip.

In another aspect, the present invention relates to a method of stratifying patients suffering from PDAC into treatment cohorts, the method comprising the steps of: (a) *in vitro* determination of the expression and/or the presence of (i) keratin 81, (ii) vimentin, (iii) one or more transcription factors selected from HNF-1A, HNF-1B, FOXA2 (HNF3B), FOXA3 (HNF3G), HNF4G, and ONECUT1 (HNF6), (iv) one or more HNF-1A target genes, particularly HNF-1A target genes listed in Table 2, and (v) cadherin17 (CDH17), (b) identifying the PDAC subtype based on the expression and/or the presence of the selected proteins, and (c) stratifying PDAC patients into drug treatment cohorts based on the PDAC subtype they are suffering from.

In particular embodiments, the method comprises the step of (a) *in vitro* determination of the expression and/or the presence of (i) keratin 81 or vimentin, particularly of keratin 81, and (ii) HNF-1A or HNF-1B, particularly HNF-1A,

In another aspect, the present disclosure relates to a method of stratifying patients suffering from PDAC into cohorts, the method comprising the steps of: (a) establishing an *in vitro* gene expression profile for the determination of the Src inhibitor sensitivity signature in cells of a patient sample containing pancreatic tissue, (b) identifying the PDAC subtype based on the gene expression profile, and (c) stratifying PDAC patients into drug treatment cohorts based on the PDAC subtype they are suffering from.

In another aspect, the present invention relates to a method of evaluating the prognosis of patients suffering from PDAC, the method comprising the steps of: (a) *in vitro* determination of (i) the expression and/or the presence of (i) keratin 81, (ii) vimentin, (iii) one or more transcription factors selected from HNF-1A, HNF-1B, FOXA2 (HNF3B), FOXA3 (HNF3G), HNF4G, and ONECUT1 (HNF6), (iv) one or more HNF-1A target genes, particularly HNF-1A target genes listed in Table 2, and (v) cadherin17 (CDH17), (b) identifying the PDAC subtype based on the expression and/or the presence of the selected proteins, and (c) stratifying PDAC patients into prognostic cohorts based on the PDAC subtype they are suffering from.

In particular embodiments, the method comprises the step of (a) *in vitro* determination of the expression and/or the presence of (i) keratin 81 or vimentin, particularly of keratin 81, and (ii) HNF-1A or HNF-1B, particularly HNF-1A,

In another aspect, the present invention relates to a method of evaluating the prognosis of patients suffering from PDAC, the method comprising the steps of: (a) establishing an *in vitro* gene expression profile for the determination of the Src inhibitor sensitivity signature in cells of a patient sample containing pancreatic tissue, (b) identifying the PDAC subtype based on the gene expression profile, and (c) stratifying PDAC patients into prognostic cohorts based on the PDAC subtype they are suffering from.

In the context of the present invention, the term "stratifying" or "stratification" relates to the identification of a group of patients with shared "biological" characteristics by using molecular and biochemical diagnostic testing to select the optimal management for the patients.

In a particular embodiment, the expression level is determined on mRNA level by quantitative real-time polymerase chain reaction (qRT-PCR).

In another embodiment, the expression level is determined on protein level based on detection of antibody binding to the selected proteins.

In particular embodiments, said cells are obtained by purifying cancer cells from said patient sample, particularly wherein the purifying step comprises flow sorting or laser capture microdissection.

In a particular embodiment, said patient sample is a tumor biopsy.

In a particular embodiment, said tumor biopsy is obtained by fine needle aspiration.

In a particular embodiment, the patient sample is selected from blood, serum, and plasma. In a particular embodiment, the patient sample is a collection of circulating tumor cells (CTCs), particularly isolated from the blood of a patient. In particular embodiments, the CTCs are isolated by apheresis.

In particular embodiments, the patient sample originates from a resectable PDAC.

### EXAMPLES

### Example 1: Identification of PDAC subtype-specific markers

Subtype-specific gene expression analysis was performed using primary xenografts, proprietary stable PDAC cell lines that retain the gene-expression pattern associated with the original tumor subtype and thus allow for molecular characterization of the classical, quasi-mesenchymal, and exocrine-like PDAC subtype for the identification of subtype-specific biomarkers, and cell line-derived tumors. Genes showing strong (> 5 fold) differential subtype-specific expression in the gene expression analysis were included in the screen for subtype-specific marker proteins. Since immunohistochemical markers are most useful for clinical settings, this initial candidate list was refined using the Protein Atlas database. In addition a search for subtype-specific expression of transcription factors using the GSEA motif module was carried out. This revealed an enrichment of genes containing binding sites for the transcription factor HNF-1 exclusively in the exocrine-like subtype, which was therefore included in the final candidate list. Expression of all candidates was tested immunohistochemically on sections of the entire panel of cell line-derived xenografts. Tumor specimen were fixed in 10% formalin overnight and embedded in paraffin. For immunohistochemistry, slides were de-paraffinized and rehydrated. Antigens were retrieved by boiling in a steam pot at pH 6 (Dako target retrieval solution, Dako, Glostrup) for 15 min, allowed to cool for 30 min and washed in distilled water. Nonspecific binding was blocked using the Linaris Avidin/Biotin blocking Kit (Vector Labs, Burlingame) according to the manufacturer's instructions. Slides were incubated with primary antibodies for 30 min, rinsed in PBS-T (PBS with 0.5% Tween-20), incubated for 20 min with the appropriate secondary antibody using the Dako REAL Detection System and rinsed in PBS-T. After blocking of endogenous peroxidase and incubation with Streptavidin HRP (20 min at RT), slides were developed with AEC (Dako) and counterstained with Hematoxylin. Candidate marker proteins were only selected, if the respective antibodies stained a specific subset of the PDAC specimens which therefore proved useful for the discrimination and classification of the three PDAC subtypes. The antibody against HNF-1A specifically stained the nuclei of tumor cells of the exocrine-like subtype. In contrast, keratin 81 expression was exclusively detectable in tumors of the quasi-mesenchymal subtype. No specific marker could be identified for the classical type that could later be shown to be predictive when analyzing patient samples. Thus, PDACs of the classical subtype can be characterized by the absence of keratin 81 and HNF-1A expression.

Primary antibodies against the selected marker proteins were used at the following dilutions: HNF-1A (H-205, Santa Cruz) 1:50, keratin 81 (36-Z, Santa Cruz) 1:100, and Vimentin (Clone V9, DAKO). All antibodies were diluted in Dako antibody diluent.

### Example 2: Prediction of Src-Inhibitor sensitivity using mRNA expression

Total RNA was isolated from PDAC cell lines or tumor xenografts using the miRNAeasy kit (Qiagen, Hilden). Gene expression analysis was performed with the Illumina BeadChip Technology (HumanHT-12). The resulting normalized gene list for each sample was sorted according to the detected signal. Genes with the highest signal were at the top and the rest sorted in descending order. This ranked gene list was used as input for the GSEA-Algorithm (Subramanian et al., loc. cit.). Each ranked list was compared separately against the sensitivity predictor signature (SRC-SP) described in Table 1, and the FDR calculated. A FDR cut-off value of 0.200 was determined to be optimal for accurate classification of samples into either Src-inhibitor sensitive or resistant. Samples resulting in a FDR < 0.200 are predicted to be sensitive, while a FDR > 0.200 predicts resistance.

### Example 3: Identification of HNF-1 target genes overexpressed in the exocrine-like subtype

We identified target genes of the transcription factor HNF-1A that are overexpressed in the exocrine-like subtype. These genes could be used as further markers for the exocrine-like subtype.

Total RNA was isolated from different PACO lines at early passage and late passage (80 % confluent) or tumor tissue (30 mg) using the miRNeasy kit (Qiagen, Hilden). Gene expression analysis was performed using the Illumina BeadChip Technology (HumanHT-12v4). For analysis of differential gene expression and clustering the TM4 Microarray Software Suite was employed (Saeed, A. I., Sharov, V., White, J., Li, J., Liang, W., Bhagabati, N., Braisted, J., Klapa, M., Currier, T., Thiagarajan, M., et al. (2003). TM4: a free, open-source system for microarray data management and analysis. BioTechniques 34, 374-378). Gene set enrichment analysis on normalized data was conducted as described previously (Subramanian et al., loc. cit.). The samples were tested against sets of genes that contain putative binding-sites for HNF-1A. The gene sets tested are contained in the MSigDatabase (http://www.broadinstitute.org/gsea/msigdb/index.jsp) and are available under the accession numbers M12675, M11442, M14593 and M15429. Genes that were contained in the core enrichment enriched as determined by the GSEA algorithm (Saeed et al., loc. cit.) were included in the signature of HNF-1A target genes expressed in the exocrine-like subtype and are listed in Table 2.

### Example 4: Determination of tumor subtype by immunohistochemistry

For formalin-fixed and paraffin-embedded tissue samples, sections of 3 µm - 5 µm were de-paraffinized and rehydrated. Antigens were retrieved by boiling in a steam pot at pH 6 (Dako target retrieval solution, Dako, Glostrup) for 15 min, allowed to cool for 30 min and washed in distilled water. Nonspecific binding was blocked using the Linaris Avidin/Biotin blocking Kit (Vector Labs, Burlingame) according to the manufacturer's instructions. Slides were incubated with primary antibodies for 30 min, rinsed in PBS-T (PBS with 0.5% Tween-20), incubated for 20 min with the appropriate secondary antibody using the Dako REAL Detection System and rinsed in PBS-T. After blocking of endogenous peroxidase and incubation with Streptavidin HRP (20 min at RT), slides were developed with AEC (Dako) and counterstained with Hematoxylin. The staining is performed on two separate sections or two separate areas on a section for HNF-1A and keratin 81, respectively.

**Evaluation of staining:** Only tumor cells are considered in the evaluation of the staining for subtype assignment. 500 tumor cells are evaluated in each specimen. A signal is considered positive if the observed signal can be clearly distinguished from the background staining observed with an isotype control antibody on a comparable specimen. The specimen is considered positive for keratin 81 if least one tumor cell shows a clearly detectable intracellular signal. The specimen is considered positive for HNF-1A if at least one of the tumor cells shows a clearly detectable nuclear staining. The subtype is then determined as follows:
Keratin 81 positive/HNF-1A negative: **quasi-mesenchymal subtype**
Keratin 81 negative/HNF-1A positive: **exocrine-like subtype**
Keratin 81 negative/HNF-1A negative: **classical subtype**
Keratin 81 positive/HNF-1A positive: undetermined

### Example 5: Correlation of marker expression with patient survival in a tissue microarray

Stratification of patients combined with subtype-specific therapeutic approaches is becoming increasingly important and has already been shown to improve the efficacy of treatments in several types of cancer. However, until now attempts to stratify PDAC patients into clinically meaningful groups have yielded mixed results (Stathis, A., and Moore, M. J. (2010). Advanced pancreatic carcinoma: current treatment and future challenges. Nat Rev Clin Oncol 7, 163-172). This could in part be attributed to the presence of at least three subtypes, complicating single-marker approaches. With a set of two markers we can unambiguously identify all three PDAC subtypes in the PACO model. Application of those markers to a cohort of 258 PDAC patients confirmed a non-overlapping staining; suggesting the robustness of our two marker-set when applied to patients groups. This allowed us to stratify a large cohort of patients into three groups, HNF1A positive (exocrine-like), keratine 81 (KRT81) positive (QM-PDA) and double-negative (classical). We found a significant difference of overall survival between the three groups. KRT81-positive patients had the worst prognosis (mean OS = 16.5 months), while HNF-1A positive patients had the best outcome (mean OS = 43.5 months) and the double negative were intermediate (mean OS = 26.3 months). Thus, our markers allow for the first time for a clinically meaningful stratification of PDAC patients and can be easily incorporated into routine diagnosis providing the possibility for stratification of PDAC patients to guide current and novel treatments.

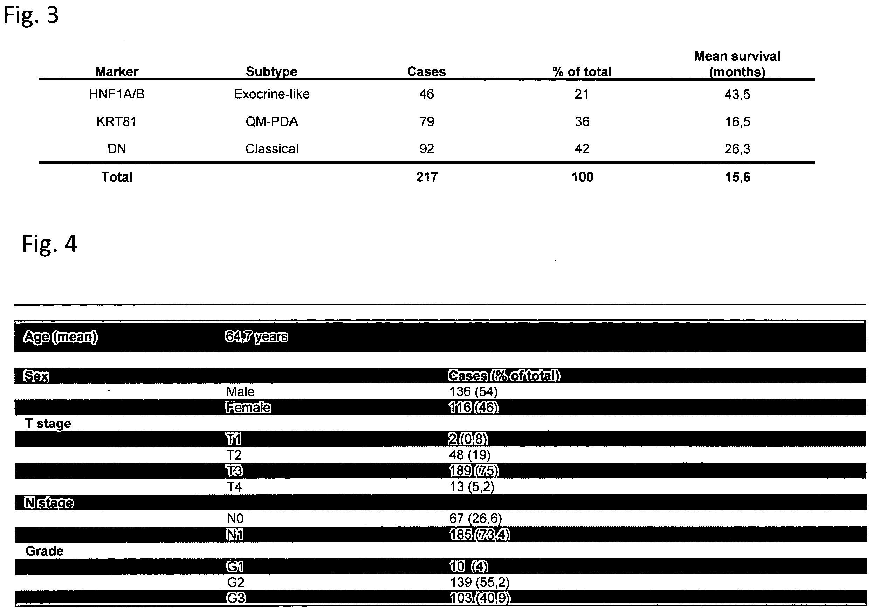

The tissue microarray was constructed from patients that received partial pancreatoduodenectomy for PDAC between 1991 and 2006 at the Charité University Hospital Berlin. The use of this tumor cohort for biomarker analysis has been approved by the Charité University ethics committee (EA1/06/2004). Patient characteristics are summarized in Figure 4.

Formalin-fixed and paraffin-embedded tissue samples were used to generate tissue microarrays as described previously (Weichert, W., Roske, A., Gekeler, V., Beckers, T., Ebert, M. P., Pross, M., Dietel, M., Denkert, C., and Rocken, C. (2008). Association of patterns of class I histone deacetylase expression with patient prognosis in gastric cancer: a retrospective analysis. The Lancet Oncology 9, 139-148). Briefly, three morphologically representative regions of the paraffin 'donor' blocks were chosen. Three tissue cylinders of 0.6 mm diameter representing these areas were punched from each sample and precisely arrayed into a new 'recipient' paraffin block using a customer built instrument (Beecher Instruments, Silver Spring, MD, USA).

**Table 1: List of genes contained in the SRC-SP predictor**

| **Gene-Symbol** | **Refseq ID** | **Definition** |
|---|---|---|
| IFI44L | NM_006820.1 | Homo sapiens interferon-induced protein 44-like (IFI44L), mRNA. |
| IFI6 | NM_022872.2 | Homo sapiens interferon, alpha-inducible protein 6 (IFI6), transcript variant 2, mRNA. |
| MX1 | NM_002462.2 | Homo sapiens myxovirus (influenza virus) resistance 1, interferon-inducible protein p78 (mouse) (MX1), mRNA. |
| OAS2 | NM_016817.2 | Homo sapiens 2'-5'-oligoadenylate synthetase 2, 69/71 kDa (OAS2), transcript variant 1, mRNA. |
| RARRES3 | NM_004585.3 | Homo sapiens retinoic acid receptor responder (tazarotene induced) 3 (RARRES3), mRNA. |
| IFI44 | NM_006417.3 | Homo sapiens interferon-induced protein 44 (IF144), mRNA. |
| IFIT1 | NM_001548.3 | Homo sapiens interferon-induced protein with tetratricopeptide repeats 1 (IFIT1), transcript variant 2, mRNA. |
| IFIT3 | NM_001031683.1 | Homo sapiens interferon-induced protein with tetratricopeptide repeats 3 (IFIT3), mRNA. |
| IFIT3 | NM_001549.2 | Homo sapiens interferon-induced protein with tetratricopeptide repeats 3 (IFIT3), mRNA. |
| IFI6 | NM_022873.2 | Homo sapiens interferon, alpha-inducible protein 6 (IFI6), transcript variant 3, mRNA. |
| ISG15 | NM_005101.1 | Homo sapiens ISG15 ubiquitin-like modifier (ISG15), mRNA. |
| IFIT2 | NM_001547.4 | Homo sapiens interferon-induced protein with tetratricopeptide repeats 2 (IFIT2), mRNA. |
| OASL | NM_198213.1 | Homo sapiens 2'-5'-oligoadenylate synthetase-like (OASL), transcript variant 2, mRNA. |
| IFI27 | NM_005532.3 | Homo sapiens interferon, alpha-inducible protein 27 (IF127), transcript variant 2, mRNA. |
| EPSTI1 | NM_033255.2 | Homo sapiens epithelial stromal interaction 1 (breast) (EPSTI1), transcript variant 2, mRNA. |
| APOBEC3G | NM_021822.1 | Homo sapiens apolipoprotein B mRNA editing enzyme, catalytic polypeptide-like 3G (APOBEC3G), mRNA. |
| HERC5 | NM_016323.2 | Homo sapiens hect domain and RLD 5 (HERC5), mRNA. |
| BST2 | NM_004335.2 | Homo sapiens bone marrow stromal cell antigen 2 (BST2), mRNA. |
| OAS3 | NM_006187.2 | Homo sapiens 2'-5'-oligoadenylate synthetase 3, 100kDa (OAS3), mRNA. |
| APOBEC3G | NM_021822.1 | Homo sapiens apolipoprotein B mRNA editing enzyme, catalytic polypeptide-like 3G (APOBEC3G), mRNA. |
| CXCL10 | NM_001565.2 | Homo sapiens chemokine (C-X-C motif) ligand 10 (CXCL10), mRNA. |
| XAF1 | NM_199139.1 | Homo sapiens XIAP associated factor 1 (XAF1), transcript variant 2, mRNA. |
| HOXB2 | NM_002145.3 | Homo sapiens homeobox B2 (HOXB2), mRNA. |
| UBE2L6 | NM_004223.3 | Homo sapiens ubiquitin-conjugating enzyme E2L 6 (UBE2L6), transcript variant 1, mRNA. |
| RSAD2 | NM_080657.4 | Homo sapiens radical S-adenosyl methionine domain containing 2 (RSAD2), mRNA. |
| CDKN2B | NM_078487.2 | Homo sapiens cyclin-dependent kinase inhibitor 2B (p15, inhibits CDK4) (CDKN2B), transcript variant 2, mRNA. |
| IRF7 | NM_004029.2 | Homo sapiens interferon regulatory factor 7 (IRF7), transcript variant b, mRNA. |
| ITGB2 | NM_000211.1 | Homo sapiens integrin, beta 2 (antigen CD18 (p95), lymphocyte function-associated antigen 1; macrophage antigen 1 (mac-1) beta subunit) (ITGB2), mRNA. |
| SAMD9L | NM_152703.2 | Homo sapiens sterile alpha motif domain containing 9-like (SAMD9L), mRNA. |
| ERAP2 | NM_022350.2 | Homo sapiens endoplasmic reticulum aminopeptidase 2 (ERAP2), mRNA. |

**Table 2: List of HNF-1 target genes that are expressed in Exocrine-like PDAC**

| SLC5A1 | MSX2 | TINAG |
|---|---|---|
| FGFR4 | MMP11 | USP3 |
| ANXA13 | TLE4 | HABP2 |
| C11ORF9 | SFRS7 | CREB5 |
| TM4SF4 | SGK2 | CLDN10 |
| KIF12 | GUCA2A | PURA |
| RNASE4 | SERPINA4 | PRODH2 |
| UGT1A1 | ALB | GRB7 |
| TCEA3 | GJB1 | AGT |
| CDH17 | SLC37A4 | TTR |
| SLC4A4 | SPINK1 | SERPING1 |
| NR5A2 | SLC12A2 | MLL |
| LGALS2 | TBL1X | THAP11 |
| NR1H4 | SLC1A1 | |
| UGT1A6 | RBP4 | |
| GATA6 | ARHGAP12 | |
| NPAS2 | NDST2 | |
| NFE2L2 | EML4 | |
| FAM20C | NEO1 | |
| TBXAS1 | THRA | |
| IGFBP1 | PRDM1 | |
| CDAN1 | LPP | |
| GUCA2B | PLS3 | |
| SLC3A1 | YES1 | |
| FOXA2 | AFM | |
| STAT5B | C14ORF138 | |
| MSH5 | BACE2 | |
| APOM | UBE3A | |
| FXYD2 | LRRC19 | |
| HPN | AQP4 | |
| SEMA4G | LRRFIP2 | |
| ROBO3 | BPHL | |
| SLC39A14 | C5 | |
| SLC7A9 | CYFIP2 | |
| NFIX | SLC39A5 | |
| PDGFRA | AXIN2 | |
| RAB31P | TMEM27 | |
| GC | SULF2 | |
| ANPEP | MIA2 | |
| CRB3 | STAG2 | |

## Claims

1. In vitro method for the subtype-specific detection of pancreatic ductal adenocarcinoma (PDAC) cells in a tumor sample **characterized by** the step of detecting expression of subtype-specific markers keratin 81 and HNF-1A, either as proteins or as oligonucleotides encoding such proteins, wherein keratin 81 is the marker for PDAC cells of the quasi-mesenchymal subtype, wherein (i) HNF-1A is the marker for cells of the exocrine-like subtype, and wherein PDAC cells of the classical subtype are **characterized by** the absence of keratin 81 and HNF-1A.

2. In vitro method of claim 1, **characterized by** the step of detecting expression of subtype-specific markers, either as proteins or as oligonucleotides encoding such proteins, wherein vimentin is detected as marker for PDAC cells of the quasi-mesenchymal subtype, wherein (i) one or more transcription factors selected from HNF-1B, FOXA2 (HNF3B), FOXA3 (HNF3G), HNF4G, and ONECUT1 (HNF6), (ii) one or more target genes regulated by HNF-1A selected from SLC5A1, FGFR4, ANXA13, C11ORF9, TM4SF4, KIF12, RNASE4, UGT1A1, TCEA3, CDH17, SLC4A4, NR5A2, LGALS2, NR1H4, UGT1A6, GATA6, NPAS2, NFE2L2, FAM20C, TBXAS1, IGFBP1, CDAN1, GUCA2B, SLC3A1, FOXA2, STAT5B, MSH5, APOM, FXYD2, HPN, SEMA4G, ROBO3, SLC39A14, SLC7A9, NFIX, PDGFRA, RAB3IP, GC, ANPEP, CRB3, MSX2, MMP11, TLE4, SFRS7, SGK2, GUCA2A, SERPINA4, ALB, GJB1, SLC37A4, SPINK1, SLC12A2, TBL1X, SLC1A1, RBP4, ARHGAP12, NDST2, EML4, NEO1, THRA, PRDM1, LPP, PLS3, YES1, AFM, C14ORF138, BACE2, UBE3A, LRRC19, AQP4, LRRFIP2, BPHL, C5, CYFIP2, SLC39A5, AXIN2, TMEM27, SULF2, MIA2, STAG2, TINAG, USP3, HABP2, CREB5, CLDN10, PURA, PRODH2, GRB7, AGT, TTR, SERPING1, MLL, and THAP11, and (iii) cadherin17 (CDH17) are detected as markers for cells of the exocrine-like subtype, and wherein PDAC cells of the classical subtype are **characterized by** the absence of (i) keratin 81 and optionally vimentin, and (ii) HNF-1A and optionally HNF-1B.

3. The in vitro method of claim 1 or 2, wherein PDAC of the classical subtype are identified by determining the absence of specific expression of one or more additional biomarkers selected from (i) transcription factors selected from HNF-1B, FOXA2 (HNF3B), FOXA3 (HNF3G), HNF4G, and ONECUT1 (HNF6), (ii) one or more target genes regulated by HNF-1A selected from SLC5A1, FGFR4, ANXA13, C11ORF9, TM4SF4, KIF12, RNASE4, UGT1A1, TCEA3, CDH17, SLC4A4, NR5A2, LGALS2, NR1 H4, UGT1A6, GATA6, NPAS2, NFE2L2, FAM20C, TBXAS1, IGFBP1, CDAN1, GUCA2B, SLC3A1, FOXA2, STAT5B, MSH5, APOM, FXYD2, HPN, SEMA4G, ROBO3, SLC39A14, SLC7A9, NFIX, PDGFRA, RAB3IP, GC, ANPEP, CRB3, MSX2, MMP11, TLE4, SFRS7, SGK2, GUCA2A, SERPINA4, ALB, GJB1, SLC37A4, SPINK1, SLC12A2, TBL1X, SLC1A1, RBP4, ARHGAP12, NDST2, EML4, NEO1, THRA, PRDM1, LPP, PLS3, YES1, AFM, C14ORF138, BACE2, UBE3A, LRRC19, AQP4, LRRFIP2, BPHL, C5, CYFIP2, SLC39A5, AXIN2, TMEM27, SULF2, MIA2, STAG2, TINAG, USP3, HABP2, CREB5, CLDN10, PURA, PRODH2, GRB7, AGT, TTR, SERPING1, MLL, and THAP11, and (iii) cadherin17 (CDH17).

4. The in vitro method according to any one of claims 1 to 3, wherein said detection is based on binding of a detection antibody to an epitope of one of said subtype-specific markers.

5. The in vitro method according to claim 4, comprising the steps of:
(a) bringing said sample into contact with a detection antibody specific for one of said subtype-specific markers under conditions that allow binding of the detection antibody to said epitope;
(b) removal of excess detection antibody molecules; and
(c) detection of bound detection antibody in said sample.

6. The in vitro method according to claim 4 or 5, wherein the detection antibody is a monoclonal antibody, a polyclonal antibody, a recombinantly produced antibody, including a recombinantly produced chimeric or humanized antibody, a fully synthetic antibody, or a functional fragment of one of said antibodies.

7. The in vitro method according to any one of claims 4 to 6, wherein detection antibody binding is detected by incubation with a secondary antibody, particularly a labeled secondary antibody, more particularly wherein the secondary antibody is labeled with a marker molecule, more particularly wherein the secondary antibody is labeled with a fluorescent dye, a biologically active enzymatic label, a radioactive label, a nuclear magnetic resonance active label, a luminescent label or a chromophoric label, most particularly wherein the radioactive label is selected from the group of ³²P, ¹²⁵I, ³H, ¹⁴C, ¹⁸⁸Rh, ¹³¹I, ^{99m}Tc and ¹¹¹In.

8. The in vitro method according to any one of claims 4 to 7, wherein antibody binding is detected by immunohistological staining.

9. The in vitro method according to any one of claims 1 to 3, wherein said detection is based on determining mRNA encoding one of said subtype-specific markers, particularly by quantitative real-time polymerase chain reaction (qRT-PCR).

10. The in vitro method according to any one of claims 1 to 9, wherein said sample contains pancreatic tissue, particularly wherein said sample is obtained from a mammal, particularly a human.

11. Kit for the subtype-specific detection of PDAC cells comprising at least two reagents specific for PDAC subtype-specific markers keratin 81 and HNF-1A and optionally one or more reagents specific for PDAC subtype-specific markers selected from the list of: (i) vimentin, (ii) one or more transcription factors selected from HNF-1B, FOXA2 (HNF3B), FOXA3 (HNF3G), HNF4G, and ONECUT1 (HNF6), (iii) one or more target genes regulated by HNF-1A selected from SLC5A1, FGFR4, ANXA13, C11ORF9, TM4SF4, KIF12, RNASE4, UGT1A1, TCEA3, CDH17, SLC4A4, NR5A2, LGALS2, NR1H4, UGT1A6, GATA6, NPAS2, NFE2L2, FAM20C, TBXAS1, IGFBP1, CDAN1, GUCA2B, SLC3A1, FOXA2, STAT5B, MSH5, APOM, FXYD2, HPN, SEMA4G, ROBO3, SLC39A14, SLC7A9, NFIX, PDGFRA, RAB3IP, GC, ANPEP, CRB3, MSX2, MMP11, TLE4, SFRS7, SGK2, GUCA2A, SERPINA4, ALB, GJB1, SLC37A4, SPINK1, SLC12A2, TBL1X, SLC1A1, RBP4, ARHGAP12, NDST2, EML4, NEO1, THRA, PRDM1, LPP, PLS3, YES1, AFM, C14ORF138, BACE2, UBE3A, LRRC19, AQP4, LRRFIP2, BPHL, C5, CYFIP2, SLC39A5, AXIN2, TMEM27, SULF2, MIA2, STAG2, TINAG, USP3, HABP2, CREB5, CLDN10, PURA, PRODH2, GRB7, AGT, TTR, SERPING1, MLL, and THAP11, and (iv) cadherin17 (CDH17), wherein said reagents are selected from the list of: detection antibodies and oligonucleotide probes, and optionally further comprising at least one reagent for the detection of at least one of said reagents.

12. A method of stratifying patients suffering from PDAC into treatment cohorts, the method comprising the steps of:
(a) in vitro determination of the expression and/or the presence of keratin 81 as marker for PDAC cells of the quasi-mesenchymal subtype and of HNF-1A as marker for PDAC cells of the exocrine-like subtype, and optionally of vimentin as marker for PDAC cells of the quasi-mesenchymal subtype, and optionally of one or more markers for PDAC cells of the exocrine-like subtype selected from (i) one or more transcription factors selected from HNF-1B, FOXA2 (HNF3B), FOXA3 (HNF3G), HNF4G, and ONECUT1 (HNF6), (ii) one or more HNF-1A target genes selected from SLC5A1, FGFR4, ANXA13, C11ORF9, TM4SF4, KIF12, RNASE4, UGT1A1, TCEA3, CDH17, SLC4A4, NR5A2, LGALS2, NR1H4, UGT1A6, GATA6, NPAS2, NFE2L2, FAM20C, TBXAS1, IGFBP1, CDAN1, GUCA2B, SLC3A1, FOXA2, STAT5B, MSH5, APOM, FXYD2, HPN, SEMA4G, ROBO3, SLC39A14, SLC7A9, NFIX, PDGFRA, RAB3IP, GC, ANPEP, CRB3, MSX2, MMP11, TLE4, SFRS7, SGK2, GUCA2A, SERPINA4, ALB, GJB1, SLC37A4, SPINK1, SLC12A2, TBL1X, SLC1A1, RBP4, ARHGAP12, NDST2, EML4, NEO1, THRA, PRDM1, LPP, PLS3, YES1, AFM, C14ORF138, BACE2, UBE3A, LRRC19, AQP4, LRRFIP2, BPHL, C5, CYFIP2, SLC39A5, AXIN2, TMEM27, SULF2, MIA2, STAG2, TINAG, USP3, HABP2, CREB5, CLDN10, PURA, PRODH2, GRB7, AGT, TTR, SERPING1, MLL, and THAP11, and (iii) cadherin17 (CDH17);
(b) identifying the PDAC subtype based on the expression and/or the presence of the selected proteins, wherein PDAC cells of the classical subtype are **characterized by** the absence of keratin 81 and HNF-1A;
(c) stratifying PDAC patients into drug treatment cohorts based on the PDAC subtype they are suffering from.

13. A method of evaluating the prognosis of patients suffering from PDAC, the method comprising the steps of:
(a) in vitro determination of the expression and/or the presence of keratin 81 as marker for PDAC cells of the quasi-mesenchymal subtype and of HNF-1A as marker for PDAC cells of the exocrine-like subtype, and optionally of vimentin as marker for PDAC cells of the quasi-mesenchymal subtype and optionally one or more markers for PDAC cells of the exocrine-like subtype selected from (i) one or more transcription factors selected from HNF-1B, FOXA2 (HNF3B), FOXA3 (HNF3G), HNF4G, and ONECUT1 (HNF6), (ii) one or more HNF-1A target genes selected from SLC5A1, FGFR4, ANXA13, C11ORF9, TM4SF4, KIF12, RNASE4, UGT1A1, TCEA3, CDH17, SLC4A4, NR5A2, LGALS2, NR1H4, UGT1A6, GATA6, NPAS2, NFE2L2, FAM20C, TBXAS1, IGFBP1, CDAN1, GUCA2B, SLC3A1, FOXA2, STAT5B, MSH5, APOM, FXYD2, HPN, SEMA4G, ROBO3, SLC39A14, SLC7A9, NFIX, PDGFRA, RAB3IP, GC, ANPEP, CRB3, MSX2, MMP11, TLE4, SFRS7, SGK2, GUCA2A, SERPINA4, ALB, GJB1, SLC37A4, SPINK1, SLC12A2, TBL1X, SLC1A1, RBP4, ARHGAP12, NDST2, EML4, NEO1, THRA, PRDM1, LPP, PLS3, YES1, AFM, C14ORF138, BACE2, UBE3A, LRRC19, AQP4, LRRFIP2, BPHL, C5, CYFIP2, SLC39A5, AXIN2, TMEM27, SULF2, MIA2, STAG2, TINAG, USP3, HABP2, CREB5, CLDN10, PURA, PRODH2, GRB7, AGT, TTR, SERPING1, MLL, and THAP11, and (iii) cadherin17 (CDH17);
(b) identifying the PDAC subtype based on the expression and/or the presence of the selected proteins, wherein PDAC cells of the classical subtype are **characterized by** the absence of keratin 81 and HNF-1A;
(c) stratifying PDAC patients into prognostic cohorts based on the PDAC subtype they are suffering from.

14. The method according to claim 12 or 13, wherein the expression level is determined on mRNA level by quantitative real-time polymerase chain reaction (qRT-PCR), or wherein the expression level is determined on protein level based on detection of antibody binding to the selected proteins.

15. The method according to any one of claims 12 to 14 wherein said cells are obtained by purifying cancer cells from said patient sample, particularly wherein the purifying step comprises flow sorting or laser capture microdissection.

16. The method according to any one of claims 12 to 15, wherein said patient sample is a tumor biopsy, particularly wherein said tumor biopsy is obtained by fine needle aspiration.

## Patentansprüche

1. In vitro-Methode für die Subtyp-spezifische Erkennung von PDAC-Zellen in einer Tumorprobe, **gekennzeichnet durch** den Schritt, die Expression von Subtyp-spezifischen Markern festzustellen, entweder als Proteine oder als Oligonukleotide, die solche Proteine kodieren, wobei Keratin 81 ein Marker für PDAC-Zellen des quasi-mesenchymalen Subtyps ist, wobei (i) HNF-1A ein Marker für Zellen des exokrin-ähnlichen Subtyps ist, und wobei PDAC-Zellen des klassischen Subtyps durch die Abwesenheit von Keratin 81 und HNF-1A gekennzeichnet sind.

2. In vitro-Methode nach Anspruch 1, **gekennzeichnet durch** den Schritt, die Expression von Subtyp-spezifischen Markern festzustellen, entweder als Proteine oder als Oligonukleotide, die solche Proteine kodieren, wobei Keratin 81 und Vimentin als Marker für PDAC-Zellen des quasi-mesenchymalen Subtyps festgestellt werden, wobei (i) ein oder mehrere Transkriptionsfaktoren ausgewählt aus der Liste HNF-1A, HNF-1B, FOXA2 (HNF3B), FOXA3 (HNF3G), HNF4G, und ONECUT1 (HNF6), (ii) ein oder mehrere durch HNF-1A regulierte Zielgene ausgewählt aus SLC5A1, FGFR4, ANXA13, C11ORF9, TM4SF4, KIF12, RNASE4, UGT1A1, TCEA3, CDH17, SLC4A4, NR5A2, LGALS2, NR1H4, UGT1A6, GATA6, NPAS2, NFE2L2, FAM20C, TBXAS1, IGFBP1, CDAN1, GUCA2B, SLC3A1, FOXA2, STAT5B, MSH5, APOM, FXYD2, HPN, SEMA4G, ROBO3, SLC39A14, SLC7A9, NFIX, PDGFRA, RAB3IP, GC, ANPEP, CRB3, MSX2, MMP11, TLE4, SFRS7, SGK2, GUCA2A, SERPINA4, ALB, GJB1, SLC37A4, SPINK1, SLC12A2, TBL1X, SLC1A1, RBP4, ARHGAP12, NDST2, EML4, NEO1, THRA, PRDM1, LPP, PLS3, YES1, AFM, C14ORF138, BACE2, UBE3A, LRRC19, AQP4, LRRFIP2, BPHL, C5, CYFIP2, SLC39A5, AXIN2, TMEM27, SULF2, MIA2, STAG2, TINAG, USP3, HABP2, CREB5, CLDN10, PURA, PRODH2, GRB7, AGT, TTR, SERPING1, MLL, und THAP11, und (iii) Cadherin17 (CDH17) als Marker für Zellen des exokrin-ähnlichen Subtyps festgestellt werden, und wobei PDAC-Zellen des klassischen Subtyps durch die Abwesenheit von (i) Keratin 81 und optional Vimentin und (ii) HNF-1A und optional HNF-1B gekennzeichnet sind.

3. Die in vitro-Methode nach Anspruch 1 oder 2, wobei PDAC des klassischen Subtyps durch die Abwesenheit der spezifischen Expression eines oder mehrerer zusätzlicher Biomarker ausgewählt aus (i) Transkriptionsfaktoren ausgewählt aus HNF-1B, FOXA2 (HNF3B), FOXA3 (HNF3G), HNF4G, und ONECUT1 (HNF6), (ii) einem oder mehreren Zielgenen, die von HNF-1A reguliert werden, ausgewählt aus SLC5A1, FGFR4, ANXA13, C110RF9, TM4SF4, KIF12, RNASE4, UGT1A1, TCEA3, CDH17, SLC4A4, NR5A2, LGALS2, NR1H4, UGT1A6, GATA6, NPAS2, NFE2L2, FAM20C, TBXAS1, IGFBP1, CDAN1, GUCA2B, SLC3A1, FOXA2, STAT5B, MSH5, APOM, FXYD2, HPN, SEMA4G, ROBO3, SLC39A14, SLC7A9, NFIX, PDGFRA, RAB3IP, GC, ANPEP, CRB3, MSX2, MMP11, TLE4, SFRS7, SGK2, GUCA2A, SERPINA4, ALB, GJB1, SLC37A4, SPINK1, SLC12A2, TBL1X, SLC1A1, RBP4, ARHGAP12, NDST2, EML4, NEO1, THRA, PRDM1, LPP, PLS3, YES1, AFM, C14ORF138, BACE2, UBE3A, LRRC19, AQP4, LRRFIP2, BPHL, C5, CYFIP2, SLC39A5, AXIN2, TMEM27, SULF2, MIA2, STAG2, TINAG, USP3, HABP2, CREB5, CLDN10, PURA, PRODH2, GRB7, AGT, TTR, SERPING1, MLL, und THAP11, und (iii) Cadherin17 (CDH17) identifiziert werden.

4. Die in vitro-Methode nach einem der Ansprüche 1 bis 3, wobei besagte Feststellung auf der Bindung eines Nachweisantikörpers an ein Epitop eines der genannten Subtyp-spezifischen Marker beruht.

5. Die in vitro-Methode nach Anspruch 4, umfassend die Schritte:
(a) Inkontaktbringen besagter Probe mit einem für einen der besagten Subtyp-spezifischen Marker spezifischen Antikörper unter Bedingungen, die die Bindung des Nachweisantikörpers an besagtes Epitop ermöglichen;
(b) Entfernung von überschüssigen Nachweisantikörpermolekülen; und
(c) Feststellung von gebundenem Antikörper in besagter Probe.

6. Die in vitro-Methode nach Anspruch 4 oder 5, wobei der Nachweisantikörper ein monoklonaler Antikörper, ein polyklonaler Antikörper, ein rekombinant hergestellter Antikörper, einschließlich eines rekombinant hergestellten chimären oder humanisierten Antikörpers, ein vollsynthetischer Antikörper, oder ein funktionales Fragment eines der besagten Antikörper ist.

7. Die in vitro-Methode nach einem der Ansprüche 4 bis 6, wobei die Bindung des Nachweisantikörpers durch Inkubation mit einem Sekundärantikörper nachgewiesen wird, insbesondere mit einem markierten Sekundärantikörper, insbesondere wobei der Sekundärantikörper mit einem Markermolekül markiert ist, insbesondere wobei der Sekundärantikörper mit einem Fluoreszenzfarbstoff, einem biologisch aktivem enzymatischen Marker, einer radioaktiven Sonde, einer in der kernmagnetische Resonanz aktiven Sonde, einer lumineszierenden Sonde oder einer chromophoren Sonde markiert ist, insbesondere wobei die radioaktive Sonde ausgewählt ist aus der Gruppe ³²P, ¹²⁵I, ³H, ¹⁴C, ¹⁸⁸Rh, ¹³¹I, ^{99m}Tc und ¹¹¹In.

8. Die in vitro-Methode nach einem der Ansprüche 4 bis 7, wobei die Antikörperbindung durch immunohistologische Färbung nachgewiesen wird.

9. Die in vitro-Methode nach einem der Ansprüche 1 bis 3, wobei der Nachweis auf der Bestimmung der mRNA beruht, die einen der besagten Subtyp-spezifischen Marker kodiert, insbesondere durch quantitative Echtzeit-Polymerasen-Kettenreaktion (qRT-PCR).

10. Die in vitro-Methode nach einem der Ansprüche 1 bis 9, wobei besagte Probe Pankreasgewebe enthält, insbesondere wobei besagte Probe von einem Säugetier, insbesondere einem Menschen erhalten wird.

11. Zusammenstellung für die Subtyp-spezifische Erkennung von PDAC-Zellen, die zumindest zwei Reagenzien umfasst, die spezifisch für die PDAC-Subtyp-spezifischen Marker Keratin 81 und HNF-1A sind, und optional ein oder mehrere Reagenzien, die spezifisch sind für PDAC-Subtyp-spezifische Marker ausgewählt aus der Liste: (i) Vimentin, (ii) einem oder mehreren Transkriptionsfaktoren ausgewählt aus HNF-1B, FOXA2 (HNF3B), FOXA3 (HNF3G), HNF4G, und ONECUT1 (HNF6), (iii) einem oder mehreren Zielgenen, die von HNF-1A reguliert werden, ausgewählt aus SLC5A1, FGFR4, ANXA13, C11ORF9, TM4SF4, KIF12, RNASE4, UGT1A1, TCEA3, CDH17, SLC4A4, NR5A2, LGALS2, NR1H4, UGT1A6, GATA6, NPAS2, NFE2L2, FAM20C, TBXAS1, IGFBP1, CDAN1, GUCA2B, SLC3A1, FOXA2, STAT5B, MSH5, APOM, FXYD2, HPN, SEMA4G, ROBO3, SLC39A14, SLC7A9, NFIX, PDGFRA, RAB3IP, GC, ANPEP, CRB3, MSX2, MMP11, TLE4, SFRS7, SGK2, GUCA2A, SERPINA4, ALB, GJB1, SLC37A4, SPINK1, SLC12A2, TBL1X, SLC1A1, RBP4, ARHGAP12, NDST2, EML4, NEO1, THRA, PRDM1, LPP, PLS3, YES1, AFM, C14ORF138, BACE2, UBE3A, LRRC19, AQP4, LRRFIP2, BPHL, C5, CYFIP2, SLC39A5, AXIN2, TMEM27, SULF2, MIA2, STAG2, TINAG, USP3, HABP2, CREB5, CLDN10, PURA, PRODH2, GRB7, AGT, TTR, SERPING1, MLL, und THAP11, und (iv) Cadherin17 (CDH17), wobei besagte Reagenzien ausgewählt sind aus der Liste: Nachweisantikörper und Oligonukleotid-Sonden, und die optional weiter zumindest ein Reagens für die Detektion von zumindest einem der besagten Reagenzien umfasst.

12. Methode zur Stratifizierung von Patienten, die an PDAC leiden, in Behandlungskohorten, wobei die Methode die Schritte umfasst:
(a) in vitro-Bestimmung der Expression und/oder des Vorhandenseins von Keratin 81 als Marker für PDAC-Zellen des quasi-mesenchymalen Subtyps und von HNF-1A als Marker für PDAC-Zellen des exokrin-ähnlichen Subtyps, und optional von Vimentin als Marker für PDAC-Zellen des quasi-mesenchymalen Subtyps, und optional von einem oder mehreren Markern für PDAC-Zellen des exokrin-ähnlichen Subtyps ausgewählt aus (i) einem oder mehreren Transkriptionsfaktoren ausgewählt aus HNF-1A, HNF-1B, FOXA2 (HNF3B), FOXA3 (HNF3G), HNF4G, und ONECUT1 (HNF6), (ii) einem oder mehreren HNF-1A-Zielgenen ausgewählt aus SLC5A1, FGFR4, ANXA13, C11ORF9, TM4SF4, KIF12, RNASE4, UGT1A1, TCEA3, CDH17, SLC4A4, NR5A2, LGALS2, NR1H4, UGT1A6, GATA6, NPAS2, NFE2L2, FAM20C, TBXAS1, IGFBP1, CDAN1, GUCA2B, SLC3A1, FOXA2, STAT5B, MSH5, APOM, FXYD2, HPN, SEMA4G, ROBO3, SLC39A14, SLC7A9, NFIX, PDGFRA, RAB3IP, GC, ANPEP, CRB3, MSX2, MMP11, TLE4, SFRS7, SGK2, GUCA2A, SERPINA4, ALB, GJB1, SLC37A4, SPINK1, SLC12A2, TBL1X, SLC1A1, RBP4, ARHGAP12, NDST2, EML4, NEO1, THRA, PRDM1, LPP, PLS3, YES1, AFM, C14ORF138, BACE2, UBE3A, LRRC19, AQP4, LRRFIP2, BPHL, C5, CYFIP2, SLC39A5, AXIN2, TMEM27, SULF2, MIA2, STAG2, TINAG, USP3, HABP2, CREB5, CLDN10, PURA, PRODH2, GRB7, AGT, TTR, SERPING1, MLL, und THAP11, und (iii) Cadherin17 (CDH17);
(b) Identifizierung des PDAC-Subtyps beruhend auf der Expression und/oder des Vorhandenseins der ausgewählten Proteine, wobei PDAC-Zellen des klassischen Subtyps durch die Abwesenheit von Keratin 81 und HNF-1A gekennzeichnet sind;
(c) Stratifizierung der PDAC-Patienten in Kohorten für die Wirkstoffbehandlung beruhend auf dem PDAC-Subtyp, unter dem sie leiden.

13. Methode zur Beurteilung der Prognose eines unter PDAC leidenden Patienten, wobei die Methode die Schritte umfasst:
(a) in vitro-Bestimmung der Expression und/oder des Vorhandenseins von Keratln 81 als Marker für PDAC-Zellen des quasi-mesenchymalen Subtyps und von HNF-1A als Marker für PDAC-Zellen des exokrin-ähnlichen Subtyps, und optional von Vimentin als Marker für PDAC-Zellen des quasi-mesenchymalen Subtyps und optional von einem oder mehreren Marker für PDAC-Zellen des exokrin-ähnlichen Subtyps ausgewählt aus (i) einem oder mehreren Transkriptionsfaktoren ausgewählt aus HNF-1A, HNF-1B, FOXA2 (HNF3B), FOXA3 (HNF3G), HNF4G, und ONECUT1 (HNF6), (ii) einem oder mehreren HNF-1A-Zielgenen ausgewählt aus SLC5A1, FGFR4, ANXA13, C11ORF9, TM4SF4, KIF12, RNASE4, UGT1A1, TCEA3, CDH17, SLC4A4, NR5A2, LGALS2, NR1H4, UGT1A6, GATA6, NPAS2, NFE2L2, FAM20C, TBXAS1, IGFBP1, CDAN1, GUCA2B, SLC3A1, FOXA2, STAT5B, MSH5, APOM, FXYD2, HPN, SEMA4G, ROBO3, SLC39A14, SLC7A9, NFIX, PDGFRA, RAB3IP, GC, ANPEP, CRB3, MSX2, MMP11, TLE4, SFRS7, SGK2, GUCA2A, SERPINA4, ALB, GJB1, SLC37A4, SPINK1, SLC12A2, TBL1X, SLC1A1, RBP4, ARHGAP12, NDST2, EML4, NEO1, THRA, PRDM1, LPP, PLS3, YES1, AFM, C14ORF138, BACE2, UBE3A, LRRC19, AQP4, LRRFIP2, BPHL, C5, CYFIP2, SLC39A5, AXIN2, TMEM27, SULF2, MIA2, STAG2, TINAG, USP3, HABP2, CREB5, CLDN10, PURA, PRODH2, GRB7, AGT, TTR, SERPING1, MLL, und THAP11, und (iii) Cadherin17 (CDH17);
(b) Identifizierung des PDAC-Subtyps beruhend auf der Expression und/oder des Vorhandenseins der ausgewählten Proteine, wobei PDAC-Zellen des klassischen Subtyps durch die Abwesenheit von Keratin 81 und HNF-1A gekennzeichnet sind;
(c) Stratifizierung der PDAC-Patienten in prognostische Kohorten beruhend auf dem PDAC-Subtyp, unter dem sie leiden.

14. Die Methode nach Anspruch 12 oder 13, wobei das Expressionsniveau auf der mRNA-Ebene durch quantitative Echtzeit-Polymerasen-Kettenreaktion (qRT-PCR) bestimmt wird, oder wobei das Expressionsniveau auf der Protein-Ebene basierend auf dem Nachweis von Antikörperbindung an die ausgewählten Protein bestimmt wird.

15. Die Methode nach einem der Ansprüche 12 bis 14, wobei besagte Zellen erhalten werden, indem Krebszellen aus besagter Patientenprobe aufgereinigt werden, insbesondere wobei der Aufreinigungssschritt eine Durchflusssortierung oder Laser-Capture-Mikrodissektion umfasst.

16. Die Methode nach einem der Ansprüche 12 bis 15, wobei besagte Patientenprobe eine Tumorbiopsie ist, insbesondere wobei besagte Tumorbiopsie durch Feinnadelaspiration erhalten wird.

## Revendications

1. Procédé in vitro pour la détection spécifique de sous-type de cellules d'adénocarcinome canalaire pancréatique dans un échantillon de tumeur **caractérisé par** l'étape de détection de l'expression des marqueurs spécifiques de sous-type kératine 81 et HNF-1A, sous forme de protéines ou sous forme d'oligonucléotides codant de telles protéines, où la kératine 81 est le marqueur pour les cellules de PDAC du sous-type quasi-mésenchymateux, où (i) HNF-1A est le marqueur pour les cellules du sous-type analogue à exocrine, et où les cellules de PDAC du sous-type classique sont **caractérisées par** l'absence de kératine 81 et de HNF-1A.

2. Procédé in vitro selon la revendication 1, **caractérisé par** l'étape de détection de l'expression de marqueurs spécifiques de sous-type, sous forme de protéines ou sous forme d'oligonucléotides codant de telles protéines, où la vimentine est détectée comme marqueur pour les cellules de PDAC du sous-type quasi-mésenchymateux, où (i) un ou plusieurs facteurs de transcription choisis parmi HNF-1B, FOXA2 (HNF3B), FOXA3 (HNF3G), HNF4G et ONECUT1 (HNF6), (ii) un ou plusieurs gènes cibles régulés par HNF-1A choisis parmi SLC5A1, FGFR4, ANXA13, C11ORF9, TM4SF4, KIF12, RNASE4, UGT1A1, TCEA3, CDH17, SLC4A4, NR5A2, LGALS2, NR1H4, UGT1A6, GATA6, NPAS2, NFE2L2, FAM20C, TBXAS1, IGFBP1, CDAN1, GUCA2B, SLC3A1, FOXA2, STAT5B, MSH5, APOM, FXYD2, HPN, SEMA4G, ROBO3, SLC39A14, SLC7A9, NFIX, PDGFRA, RAB3IP, GC, ANPEP, CRB3, MSX2, MMP11, TLE4, SFRS7, SGK2, GUCA2A, SERPINA4, ALB, GJB1, SLC37A4, SPINK1, SLC12A2, TBL1X, SLC1A1, RBP4, ARHGAP12, NDST2, EML4, NEO1, THRA, PRDM1, LPP, PLS3, YES1, AFM, C14ORF138, BACE2, UBE3A, LRRC19, AQP4, LRRFIP2, BPHL, C5, CYFIP2, SLC39A5, AXIN2, TMEM27, SULF2, MIA2, STAG2, TINAG, USP3, HABP2, CREB5, CLDN10, PURA, PRODH2, GRB7, AGT, TTR, SERPING1, MLL et THAP11, et (iii) la cadhérine17 (CDH17) sont détectés comme marqueurs pour les cellules du sous-type analogue à exocrine, et où les cellules de PDAC du sous-type classique sont **caractérisées par** l'absence de (i) kératine 81 et éventuellement de vimentine, et (ii) de HNF-1A et éventuellement de HNF-1B.

3. Procédé in vitro selon la revendication 1 ou 2, où les PDAC du sous-type classique sont identifiés en déterminant l'absence d'expression spécifique d'un ou plusieurs biomarqueurs supplémentaires choisis parmi (i) les facteurs de transcription choisis parmi HNF-1B, FOXA2 (HNF3B), FOXA3 (HNF 3G), HNF4G et ONECUT1 (HNF6), (ii) un ou plusieurs gènes cibles régulés par HNF-1A choisis parmi SLC5A1, FGFR4, ANXA13, C11ORF9, TM4SF4, KIF12, RNASE4, UGT1A1, TCEA3, CDH17, SLC4A4, NR5A2, LGALS2, NR1H4, UGT1A6, GATA6, NPAS2, NFE2L2, FAM20C, TBXAS1, IGFBP1, CDAN1, GUCA2B, SLC3A1, FOXA2, STAT5B, MSH5, APOM, FXYD2, HPN, SEMA4G, ROBO3, SLC39A14, SLC7A9, NFIX, PDGFRA, RAB3IP, GC, ANPEP, CRB3, MSX2, MMP11, TLE4, SFRS7, SGK2, GUCA2A, SERPINA4, ALB, GJB1, SLC37A4, SPINK1, SLC12A2, TBL1X, SLC1A1, RBP4, ARHGAP12, NDST2, EML4, NEO1, THRA, PRDM1, LPP, PLS3, YES1, AFM, C14ORF138, BACE2, UBE3A, LRRC19, AQP4, LRRFIP2, BPHL, C5, CYFIP2, SLC39A5, AXIN2, TMEM27, SULF2, MIA2, STAG2, TINAG, USP3, HABP2, CREB5, CLDN10, PURA, PRODH2, GRB7, AGT, TTR, SERPING1, MLL et THAP11, et (iii) la cadhérine 17 (CDH17).

4. Procédé in vitro selon l'une quelconque des revendications 1 à 3, où ladite détection est basée sur la liaison d'un anticorps de détection à un épitope de l'un desdits marqueurs spécifiques de sous-type.

5. Procédé in vitro selon la revendication 4, comprenant les étapes de :
(a) mise en contact dudit échantillon avec un anticorps de détection spécifique de l'un desdits marqueurs spécifiques de sous-type dans des conditions qui permettent la liaison de l'anticorps de détection audit épitope ;
(b) retrait des molécules d'anticorps de détection en excès ; et
(c) détection d'anticorps de détection lié dans ledit échantillon.

6. Procédé in vitro selon la revendication 4 ou 5, où l'anticorps de détection est un anticorps monoclonal, un anticorps polyclonal, un anticorps produit de manière recombinante, incluant un anticorps chimérique ou humanisé produit de manière recombinante, un anticorps totalement synthétique, ou un fragment fonctionnel de l'un desdits anticorps.

7. Procédé in vitro selon l'une quelconque des revendications 4 à 6, où la liaison d'un anticorps de détection est détectée par incubation avec un anticorps secondaire, en particulier un anticorps secondaire étiqueté, plus particulièrement où l'anticorps secondaire est étiqueté avec un molécule marqueur, plus particulièrement où l'anticorps secondaire est étiqueté avec un colorant fluorescent, une étiquette enzymatique biologiquement active, une étiquette radioactive, une étiquette active en résonance magnétique nucléaire, une étiquette luminescente ou une étiquette chromophore, tout particulièrement où l'étiquette radioactive est choisie dans le groupe de ³²P,¹²⁵I, ³H, ¹⁴C, ¹⁸⁸Rh, ¹³¹I ^{99m}Tc et ¹¹¹In_{.}

8. Procédé in vitro selon l'une quelconque des revendications 4 à 7, où la liaison d'un anticorps est détectée par coloration immunohistologique.

9. Procédé in vitro selon l'une quelconque des revendications 1 à 3, où ladite détection est basée sur la détermination d'ARNm codant l'un desdits marqueurs spécifiques de sous-type, en particulier par réaction en chaîne par polymérase en temps réel quantitative (qRT-PCR).

10. Procédé in vitro selon l'une quelconque des revendications 1 à 9, où ledit échantillon contient du tissu pancréatique, en particulier où ledit échantillon est obtenu auprès d'un mammifère, en particulier d'un humain.

11. Kit pour la détection spécifique de sous-type de cellules de PDAC comprenant au moins deux réactifs spécifiques des marqueurs spécifiques de sous-type kératine 81 et HNF-1A et éventuellement un ou plusieurs réactifs spécifiques de marqueurs spécifiques de sous-type de PDAC choisis dans la liste de: (i) la vimentine, (ii) un ou plusieurs facteurs de transcription choisis parmi HNF-1B, FOXA2 (HNF3B), FOXA3 (HNF3G), HNF4G et ONECUT1 (HNF6), (iii) un ou plusieurs gènes cibles régulés par HNF-1A choisis parmi SLC5A1, FGFR4, ANXA13, C11ORF9, TM4SF4, KIF12, RNASE4, UGT1A1, TCEA3, CDH17, SLC4A4, NR5A2, LGALS2, NR1H4, UGT1A6, GATA6, NPAS2, NFE2L2, FAM20C, TBXAS1, IGFBP1, CDAN1, GUCA2B, SLC3A1, FOXA2, STAT5B, MSH5, APOM, FXYD2, HPN, SEMA4G, ROBO3, SLC39A14, SLC7A9, NFIX, PDGFRA, RAB3IP, GC, ANPEP, CRB3, MSX2, MMP11, TLE4, SFRS7, SGK2, GUCA2A, SERPINA4, ALB, GJB1, SLC37A4, SPINK1, SLC12A2, TBL1X, SLC1A1, RBP4, ARHGAP12, NDST2, EML4, NEO1, THRA, PRDM1, LPP, PLS3, YES1, AFM, C14ORF138, BACE2, UBE3A, LRRC19, AQP4, LRRFIP2, BPHL, C5, CYFIP2, SLC39A5, AXIN2, TMEM27, SULF2, MIA2, STAG2, TINAG, USP3, HABP2, CREB5, CLDN10, PURA, PRODH2, GRB7, AGT, TTR, SERPING1, MLL et THAP11, et (iv) la cadhérine 17 (CDH17), où lesdits réactifs sont choisis dans la liste de : les anticorps de détection et les sondes oligonucléotidiques, et éventuellement comprenant en outre au moins un réactif pour la détection d'au moins l'un desdits réactifs.

12. Procédé de stratification de patients souffrant de PDAC en cohortes de traitement, le procédé comprenant les étapes de :
(a) détermination in vitro de l'expression et/ou de la présence de kératine 81 comme marqueur pour les cellules de PDAC du sous-type quasi-mésenchymateux et de HNF-1A comme marqueur pour les cellules de PDAC du sous-type analogue à exocrine, et éventuellement de vimentine comme marqueur pour les cellules de PDAC du sous-type quasi-mésenchymateux, et éventuellement d'un ou plusieurs marqueurs pour les cellules de PDAC du sous-type analogue à exocrine choisis parmi (i) un ou plusieurs facteurs de transcription choisis parmi HNF-1B, FOXA2 (HNF3B), FOXA3 (HNF3G), HNF4G et ONECUT1 (HNF6), (ii) un ou plusieurs gènes cibles régulés par HNF-1A choisis parmi SLC5A1, FGFR4, ANXA13, C11ORF9, TM4SF4, KIF12, RNASE4, UGT1A1, TCEA3, CDH17, SLC4A4, NR5A2, LGALS2, NR1H4, UGT1A6, GATA6, NPAS2, NFE2L2, FAM20C, TBXAS1, IGFBP1, CDAN1, GUCA2B, SLC3A1, FOXA2, STAT5B, MSH5, APOM, FXYD2, HPN, SEMA4G, ROBO3, SLC39A14, SLC7A9, NFIX, PDGFRA, RAB3IP, GC, ANPEP, CRB3, MSX2, MMP11, TLE4, SFRS7, SGK2, GUCA2A, SERPINA4, ALB, GJB1, SLC37A4, SPINK1, SLC12A2, TBL1X, SLC1A1, RBP4, ARHGAP12, NDST2, EML4, NEO1, THRA, PRDM1, LPP, PLS3, YES1, AFM, C14ORF138, BACE2, UBE3A, LRRC19, AQP4, LRRFIP2, BPHL, C5, CYFIP2, SLC39A5, AXIN2, TMEM27, SULF2, MIA2, STAG2, TINAG, USP3, HABP2, CREB5, CLDN10, PURA, PRODH2, GRB7, AGT, TTR, SERPING1, MLL et THAP11, et (iii) la cadhérine 17 (CDH17) ;
(b) identification du sous-type de PDAC sur la base de l'expression et/ou de la présence des protéines sélectionnées, où les cellules de PDAC du sous-type classique sont **caractérisées par** l'absence de kératine 81 et de HNF-1A ;
(c) stratification de patients atteints de PDAC en cohortes de traitement médicamenteux sur la base du sous-type de PDAC dont ils souffrent.

13. Procédé d'évaluation du pronostic de patients souffrant de PDAC, le procédé comprenant les étapes de :
(a) détermination in vitro de l'expression et/ou de la présence de kératine 81 comme marqueur pour les cellules de PDAC du sous-type quasi-mésenchymateux et de HNF-1A comme marqueur pour les cellules de PDAC du sous-type analogue à exocrine, et éventuellement de vimentine comme marqueur pour les cellules de PDAC du sous-type quasi-mésenchymateux et éventuellement d'un ou plusieurs marqueurs pour les cellules de PDAC du sous-type analogue à exocrine choisis parmi (i) un ou plusieurs facteurs de transcription choisis parmi HNF-1B, FOXA2 (HNF3B), FOXA3 (HNF3G), HNF4G et ONECUT1 (HNF6), (ii) un ou plusieurs gènes cibles régulés par HNF-1A choisis parmi SLC5A1, FGFR4, ANXA13, C11ORF9, TM4SF4, KIF12, RNASE4, UGT1A1, TCEA3, CDH17, SLC4A4, NR5A2, LGALS2, NR1H4, UGT1A6, GATA6, NPAS2, NFE2L2, FAM20C, TBXAS1, IGFBP1, CDAN1, GUCA2B, SLC3A1, FOXA2, STAT5B, MSH5, APOM, FXYD2, HPN, SEMA4G, ROBO3, SLC39A14, SLC7A9, NFIX, PDGFRA, RAB3IP, GC, ANPEP, CRB3, MSX2, MMP11, TLE4, SFRS7, SGK2, GUCA2A, SERPINA4, ALB, GJB1, SLC37A4, SPINK1, SLC12A2, TBL1X, SLC1A1, RBP4, ARHGAP12, NDST2, EML4, NEO1, THRA, PRDM1, LPP, PLS3, YES1, AFM, C14ORF138, BACE2, UBE3A, LRRC19, AQP4, LRRFIP2, BPHL, C5, CYFIP2, SLC39A5, AXIN2, TMEM27, SULF2, MIA2, STAG2, TINAG, USP3, HABP2, CREB5, CLDN10, PURA, PRODH2, GRB7, AGT, TTR, SERPING1, MLL et THAP11, et (iii) la cadhérine 17 (CDH 17) ;
(b) identification du sous-type de PDAC sur la base de l'expression et/ou de la présence des protéines sélectionnées, où les cellules de PDAC du sous-type classique sont **caractérisées par** l'absence de kératine 81 et de HNF-1A ;
(c) stratification de patients atteints de PDAC en cohortes de pronostic sur la base du sous-type de PDAC dont ils souffrent.

14. Procédé selon la revendication 12 ou 13, où le niveau d'expression est déterminé au niveau de l'ARNm par réaction en chaîne par polymérase en temps réel quantitative (qRT-PCR), ou bien où le niveau d'expression est déterminé au niveau des protéines sur la base de la détection d'anticorps se liant aux protéines sélectionnées.

15. Procédé selon l'une quelconque des revendications 12 à 14 où lesdites cellules sont obtenues par purification de cellules cancéreuses à partir dudit échantillon d'un patient, en particulier où l'étape de purification comprend le tri en flux ou la microdissection par capture laser.

16. Procédé selon l'une quelconque des revendications 12 à 15, où ledit échantillon d'un patient est une biopsie de tumeur, en particulier où ladite biopsie de tumeur est obtenue par aspiration avec une aiguille fine.
